# EUROPÄISCHE PATENTANMELDUNG

(11) **EP 1 215 495 A2**
(43) Veröffentlichungstag der Anmeldung: **19.06.2002**
(21) Anmeldenummer: 01128432.0
(22) Anmeldetag: 05.12.2001
(51) Int. Cl.: G01N 33/53

(54) **Verfahren zur Erhöhung der klinischen Spezifität bei der Detektion von Tumoren und ihren Vorstufen durch simultane Messung von mindestens zwei verschiedenen molekularen Markern**

(30) Priorität: 18.12.2000 DE 10063112
(71) Anmelder: BAYER AG, 51368 Leverkusen (DE)
(72) Erfinder: Hennig, Guido, Dr., 50859 Köln (DE); Wirtz, Ralph, Dr., 50674 Köln (DE); Bohmann, Kerstin, Dr., 47798 Krefeld (DE); Schöpper, Birge, 50259 Pulheim (DE)

(57) **Zusammenfassung**

Die vorliegende Erfindung betrifft ein automatisierbares Verfahren zur verbesserten Diagnose von krankhaft veränderten Zellen durch die simultane Anfärbung von mindestens zwei verschiedenen molekularen Markern, die krankheits-assoziiert eine veränderte Genexpression aufweisen, in einer Zelle oder Teilbereichen einer Gewebeprobe durch die Verwendung von Kombinationen von Antikörpern und die Verrechnung von Signalintensitäten.

## Beschreibung

### Stand der Technik

Nach wie vor zählen Krebserkrankungen zu den häufigsten Todesursachen weltweit. Es besteht ein klinischer Bedarf sowohl an der Früherkennung und spezifischen Detektion von Krebs und Krebsvorstufen. Zudem ist eine weiterführende, detaillierte Charakterisierung des Tumors wichtig um Aussagen bezüglich Therapie und Prognose zu machen. Dies ist nützlich, da verschiedene Karzinomsubtypen unterschiedlich auf angewendete Therapiemaßnahmen reagieren. Dazu werden, z.B. bei Verdacht auf Brustkrebs, immer häufiger immunhistochemische Färbungen von molekularen Markern, wie z.B. Cytokeratine, Estrogenrezeptor, her2/neu, Ki67 oder p53 hinzugezogen (z.B. Leek *et al,* 1994, Br. J. Cancer, 69, 135-139; Mokbel *et al,* 1999, Am. J. surgery, 178, 69-72; Reiner *et al,* 1990, Cancer Res., 50, 7057-7061). Diese werden meistens auf Brustbiopsien, zunehmend jedoch auch auf Nadelpunktionen (FNAs = fine needle aspirates) durchgeführt. Auch bei der Entscheidung, ob es sich um einen Primärtumor oder eine Metastase handelt, werden molekulare Marker eingesetzt. Die Expression von Markern, die z.B. spezifisch für das primär befallene Organ sind, erleichtern dessen Identifikation.

In den letzten Jahren wurden zahlreiche Gene identifiziert, die aufgrund von Mutationen zu einer veränderten Expression ihrer Proteine führen und somit eine Rolle bei der Krebsentstehung spielen. Die korrespondierenden Proteine werden als diagnostische Marker sowohl im Serum als auch auf zellulärer Ebene nachgewiesen. Diese Proteine sind an vielfältigen physiologischen Regulationsprozessen in der Zelle, wie z.B. der Wachstumskontrolle (Proliferationsgene, z.B. Ki67, mcm-5; Onkogene und Tumorsuppressorgene, z.B. p16, EGF-Rezeptor, her2/neu, mdm-2), Apoptose (natürlicher Zelltod, z.B. p53, bcl-2), Reparatur von DNA (msh-1) und Zelladhäsion (E-Cadherin, β-catenin, APC), beteiligt. Zum Nachweis eignen sich z.B. immunhisto- und immuncytochemische Detektionsmethoden mit spezifischen Antikörpern gegen diese Proteine (van Noorden, 1986, Immuncytochemistry, Modern Methods and Applications , 2^{nd} edition, Wright, Bristol, 26-53).

Es ist vielfach gezeigt, dass ein einzelner Marker keine ausreichende Spezifität hinsichtlich der Erkennung von krankhaft veränderten Zellen erreicht, da er zum Teil auch in gesunden Zellen vorhanden ist. Dies basiert auf der Tatsache, dass diese Marker Proteine sein können, die an physiologischen Regulationsprozessen in gesunden Zellen beteiligt sind und lediglich eine überhöhte Expression ein Anzeichen für eine krankhaft veränderte Zelle ist. Williams *et al.* (1998, PNAS, 95, 14932-14937) beschreiben den DNA Replikationsmarker mcm-5, welcher 28 getestete Läsionen/Tumore von Cervix-Abstrichen (n=58) detektiert, aber 2 von 28 Normalabstrichen falsch positiv anfärbt. Sano *et al.* (Pathology Intl., 1998, 48, 580-585) beschreiben, dass der Marker p16 unspezifisch 3 von 15 normalen Cervixbiopsien anfärbt. HPV ist in nahezu 100 % aller Läsionen und Tumoren des Cervix, jedoch auch in vielen normalen Cervixepithelien, nachzuweisen, d.h. ein Test zum Nachweis von HPV weist eine hohe Sensitivität jedoch nur eine geringe Spezifität auf(z.B. Cuzick, 2000, JAMA, 283, 108-109)

In der Pathologie ist es zunehmend von Interesse mehrere Marker in einer biologischen Probe nachzuweisen. Zum einen steht zur Detektion und Charakterisierung von krankhaft veränderten Zellen oder Gewebebereichen mehr Information zur Verfügung, zum anderen können unspezifische Färbungen einzelner Marker als solche eher erkannt werden und somit Fehlaussagen vermieden werden. Traditionell werden zu diesem Zweck serielle Schnitte einer vorhandenen Biopsie bzw. mehrfache Präparationen einer Körperprobe (z.B. Gebärmutterhalsabstrich in Fixierflüssigkeit) hergestellt und mit jeweils einem bestimmten Marker gefärbt. Dieses Verfahren hat mehrere Nachteile: hoher Materialverbrauch der meist limitierten Patientenprobe und von Färbereagenzien, hoher Zeitaufwand und Kosten sowie keine Möglichkeit der eindeutigen Co-Lokalisierung von zwei oder mehreren Markern in einer Zelle oder bestimmten Arealen der Gewebeprobe. Der simultane Nachweis von zwei oder mehreren verschiedenen biologischen Markern auf einer biologischen Probe ist bereits in der Literatur beschrieben (DAKO, Practical Guide for Immunoenzymatic Double Staining Methods; Gomez *et al,* Eur. J. Histochem., 1997, 41, *255-259),* findet jedoch in der Routinediagnostik bisher keine Anwendung, da die Protokolle wenig standardisiert sind. Terhavauta *et al.* (Cytopathology, 1994, 5, 282-293) verwenden eine Doppelfärbung von p53 und Ki67 auf Biopsien von Cervix-Läsionen und können beide Marker in Basal- und Parabasalzellen von HPV positiven und negativen Läsionen z.T. in einer Zelle nachweisen. Dabei benutzen die Autoren jedoch die Information nicht, um durch Kombination der Ergebnisse eine gesichertere Aussage über das Auftreten von Tumorzellen in dieser Probe treffen zu können. Ein Ansatz zur Nutzung der Färbeinformation im Hinblick auf eine automatisierte Detektion von Tumorbereichen fehlt vollständig. Auch sind neben dieser einen keine weiteren Markerkombinationen benannt, die eine Identifizierung und Charakterisierung des Tumorbereichs ermöglichen könnten.

Weitere Doppelfärbungen auf Tumorbiopsien der Lunge sind erwähnt in Kraeft *et al* (Clin Cancer Res, 2000, 6, 434-442) sowie Oshika *et al* (Mod Pathol, 1998, 11, 1059-1063). Die dort beschriebenen Antikörperkombinationen sind vollkommen verschieden von den hier in der Erfindung zu Grunde liegenden Kombinationen. Des weiteren ist in beiden beschriebenen Publikationen zur Diagnosestellung weiterhin ein Pathologe notwendig, der die Färbung auf der Biopsie bewertet Diese Vorgehensweise ist konzeptionell inkompatibel mit einer Automatisierung des Verfahrens und wurde daher bisher nie in Betracht gezogen.

Rao *et al.* (1998, Cancer Epidemology, Biomarkers and Evolution, 7, 1027-1033) beschreiben eine Mehrfachfärbung von verschiedenen tumorassoziierten Markern (DNA-Gehalt, G-Aktin und p53) auf Nadelpunktionsmaterial (FNA, fine needle aspirates) der Brust. Sie interpretieren ihre Ergebnisse dahingehend, dass durch die Verwendung mehrerer Marker eine höhere klinische Spezifität erreicht wird, als die Betrachtung der einzelnen Marker und somit bei der Früherkennung von Brustkrebs eine wichtige Rolle spielen können. Die von den Autoren angeführte Mehrfachfärbung ist auf Nadelpunktionsmaterial der Brust beschränkt und kann auf Grund der unterschiedlichen Stoffeigenschaften nicht auf andere biologische Materialien (i.e. Biopsien, Gebärmutterhalsabstriche) übertragen werden. Des weiteren unterscheiden sich die Markerkombinationen von den hier in der Erfindung genannten Kombinationen. Es fehlt der Ansatz zur Nutzung der Färbeinformation im Hinblick auf eine automatisierte Detektion von Tumorbereichen

Eine weitere Verbesserung der momentanen klinischen Tumordiagnostik besteht sowohl in der Automatisierung der Probenpräparation und -färbung, als auch in der automatisierten Bildanalyse mit Diagnosestellung. Dies führt neben einer Zeitersparnis und einem geringeren Personalaufwand auch zu einer objektiveren und damit einheitlicheren Diagnose. Die Anfärbung von biologischen Markern in Tumorzellen oder deren Vorläufern durch Fluoreszenz oder chromogene Farbstoffe ermöglicht eine Quantifizierung und somit automatisierte Auslesung der Signale. Es gibt bereits Systeme, die eine automatisierte Durchführung von Färbeprotokollen erlauben. Es fehlt jedoch eine automatisierte Diagnose durch Bildanalyse (LeNeel *et al,* Clin Chim Acta, 1998, 278, 185-192). Am weitesten fortgeschritten ist die automatisierte Detektion von Tumorzellen und deren Vorläufern mit Hilfe morphologischer Bildinformationen bei Cervix-Abstrichen (Sawaya *et al,* Clinical Obstetrics and Gynecology, 1999, 42, 922-938; Stoler, 2000, Mod. Pathol., 13, 275-284).

Boon *et al.* (1995, Diagn. Cytopathol., 13, 423-428) nutzen neben der automatisierten morphologischen Detektion von abnormalen Zellen in Gebärmutterhalsabstrichen mit dem PAPNET System zusätzlich die immunhistochemische Anfärbung von proliferierenden Zellen mit Ki67-Antikörpern. Der Gebrauch dieses einen molekularen Markers ermöglicht jedoch keine eindeutige Unterscheidung zwischen gutartigen proliferierenden und kanzerogenen Zellen. Es wird aber in keinster Weise auf den gleichzeitigen Nachweis von mindestens zwei molekularen Markern zur Detektion von abnormalen Zellen eingegangen.

In dem Patent von Boon *et al.* (US-5544650) ist beschrieben, dass eine Färbung mit einem immunhistochemischen Marker die automatische Detektion von proliferierenden (kanzerogene und nicht-kanzerogene) Zellen in einer Probe erleichtert und in einem semi-automatischen Prozess der kontrollierende Pathologe/Cytologe entscheidet, ob es sich bei den gefärbten Zellen wirklich um kanzerogene Zellen handelt. Es werden jedoch keine bestimmten Antikörperkombinationen benannt, die eine automatisierte Detektion von abnormalen bzw. Tumorzellen ermöglichen.

Das Patent US-6005256 von McGlynn und Akkapeddi beschreibt ausführlich ein Gerät und ein Verfahren zur simultanen Detektion von mehreren fluoreszenzmarkierten Markern in einer Körperprobe, auch zur Identifikation von Krebszellen, ohne aber auf eine konkrete Anwendung in der Krebsdiagnostik einzugehen bzw. entsprechende Markerkombinationen mit erhöhter Spezifität zu benennen.

Von Ampersand Medical Systems Group (www.ampersandmedical.com) ist bekannt, dass sie ein neues Screening System für Cervix-Abstriche (InPath) entwickeln, welches neben einer eigenen Probenaufbereitung auch die Fluoreszenzdetektion von nicht genannten biologischen Markern beinhaltet.

### Aufgabe

Die vorliegende Erfindung beschreibt ein automatisierbares Verfahren mit denen krankhaft veränderte Zellen vorzugsweise Krebszellen und ihre Vorläufer, durch die Verwendung definierter Markerkombinationen bestimmt werden können.

Erfindungsgemäß wird dies durch die Gegenstände in den Patentansprüchen erreicht.

### Summarische Beschreibung / Lösung

Die vorliegende Erfindung beruht auf den Erkenntnissen der Anmelder, dass durch gleichzeitigen Nachweis von mindestens zwei bestimmten molekularen Markern, nämlich krankheits-assoziierten Veränderungen von Genexpressionen, in einer Zelle oder Gewebeprobe, die Spezifität des Nachweises von krankhaft veränderten Zellen, vorzugsweise Karzinomen und deren Vorstufen, erhöht wird und auf Grund der Detektion mehrerer Marker sowie der Kombination und Verrechnung der Signale eine spezifischere und darüber hinaus auch eine automatisierbare Detektion ermöglicht.

### Detaillierte Beschreibung:

Die Erfindung bezieht sich auf molekulare Marker, die bei Einzelnachweisen eine nicht ausreichende Spezifität hinsichtlich der Erkennung von krankhaft veränderten Zellen oder Geweben erreichen, da sie zum Teil auch in nicht krankhaft verändertem biologischen Material in ähnlicher oder unterschiedlicher Menge vorhanden sind. Dies basiert auf der Tatsache, dass diese Marker Proteine sein können, die an physiologischen Regulationsprozessen auch in gesunden Zellen beteiligt sind. Darüber hinaus kann der Nachweis des molekularen Markers aufgrund unspezifischer Kreuzreaktion des Antikörpers nicht eindeutig sein und äußert sich in der Anfärbung von Partikeln des biologischem Materials, die den molekularen Marker nicht beinhalten. Die Erfinder haben die Beobachtung gemacht, dass durch den gleichzeitigen Nachweis von mindestens zwei Markern in einer Zelle oder innerhalb eines Teilbereichs des biologischen Materials, z.B. innerhalb einer eng umgrenzten Region eines Gewebeschnittes, die mangelnde Spezifität des Einzelmarkernachweises kompensiert werden kann, so dass eine höhere Spezifität beim Nachweis von abnormalen Zellen bzw. Gewebeabschnitten gewährleistet ist. Somit ist durch die Kombination mehrerer Marker bei der Diagnostik von biologischen Proben die Aussagekraft größer als bei der Verwendung von Einzelmarkern.

Die Markerkombinationen beinhalten die Visualisierung veränderter Genexpressionen von mindestens einer der nachfolgend aufgeführten Genklassen: Onkogene, Tumorsuppressorgene, Apoptosegene, Proliferationsgene, Reparaturgene und Virusgene wie beispielhaft an Kombinationen der molekularen Marker Her2/neu, p16, p53, Ki67, MN, mdm-2, bcl-2 und EGF Rezeptor gezeigt. Bevorzugt sind folgende Kombinationen gemeint: Her2/neu und Ki67, Her2/neu und p53, her2/neu und bcl-2, her2/neu und p16, bcl-2 und Ki67, bcl-2 und p53, bcl-2 und p16, p16 und p53, p16 und Ki67.

Erfindungsgemäß werden die Erkenntnisse der Anmelder für ein Verfahren zur frühen Diagnose krankheits-assoziierter Zellen bzw. Gewebeabschnitte genutzt, das die Detektion von Kombinationen der beschriebenen molekularen Marker mit dem Ziel einer automatisierten Detektion von Karzinomen und ihrer Vorstufen sowie gegebenenfalls der Herkunft von Metastasen umfasst. Die automatisierte und spezifische Detektion von Tumorzellen kann durch folgendes Verfahren gewährleistet werden: Zum einen müssen mindestens zwei Signale in einer Zelle oder begrenzten Areal der Körperprobe vorhanden sein und zum anderen muss das Signal der beiden Marker jeweils über oder unter einer individuell definierten Intensität bzw. Schwellwert liegen. Durch die Anwendung dieser beiden Kriterien kann man gesunde Zellen ausschließen, welche z.B. einen Marker über der gesetzten Schwelle exprimieren oder beide Marker unterhalb der jeweilig definierten Signalstärke aufweisen.

Der Ausdruck "Molekularer Marker" umfasst molekulare Veränderungen von Zellen, insbesondere Veränderungen der Genexpression, die im Zusammenhang mit einer veränderten oder krankhaften Zellbeschaffenheit beobachtet worden sind. Verfahren zum Nachweis molekularer Marker umfassen jegliche Verfahren, die die Quantität oder Qualität der Marker bestimmen, vorzugsweise auf Proteinebene. Für den Nachweis auf Proteinebene bietet sich die Verwendung von Antikörpern oder anderen spezifischen Bindungsproteinen (z.B. Anti-Cullinen) an, die einen anschließenden cytochemischen oder histochemischen Nachweis mit chromogener und/oder fluoreszenter Detektion erlauben.

Der Ausdruck "gleichzeitiger Nachweis von mindestens zwei bestimmten molekularen Markern" umfasst Verfahren, die mindestens zwei Genexpressionen in einer Körperprobe, insbesondere auch in einer einzelnen Präparation der Körperprobe, erkennbar machen, so dass die Genexpressionen in Zusammenhang miteinander betrachtet werden können. Dabei ist nicht nur das Vorhandensein von mindestens zwei Signalen, sondern auch die An- oder Abwesenheit eines oder mehrerer weiterer Signale von möglicher kombinatorischer Aussagekraft. Diese weitere Information könnte zusätzlich zu der Tumorspezifität eine Aussage über die Herkunft einer Metastase beinhalten. Von Bedeutung ist dabei, dass die nachgewiesenen Marker in relativer räumlicher Nähe, insbesondere in einer Zelle, oder innerhalb eines eng umgrenzten Probenbereichs, z.B. innerhalb eines Areals eines Gewebeschnittes, detektiert werden.

Der Ausdruck "Kombination und Verrechnung der Signale" umfasst die Verknüpfung von mindestens zwei Informationsinhalten, die auf Grund des Nachweises von mindestens zwei Markern in einer Körperprobe gewonnen wurden. Zusätzlich können durch Definition von Schwellwerten der Markerintensitäten gesunde Zellen spezifisch von krankhaften Zellen unterschieden werden.

Der Ausdruck " Zelle oder Gewebeprobe" umfasst Zellen, die aus Körperproben, wie z.B. Abstriche, Sputum, Organpunktate, Biopsien, Sekrete, Liquor, Galle, Blut, Lymphflüssigkeit, Urin, Stuhl isoliert werden bzw. Gewebe, welches von Organen, wie z.B. Brust, Lunge, Darm, Haut, Cervix, Prostata und Magen entnommen wurde. Eine Gewebeprobe umfasst dabei einen Bereich funktional zusammenhängender Zellen oder benachbarte Zellen.

Der Ausdruck "krankhaft veränderte Zellen" umfasst Karzinome und deren Vorläufer, vorzugsweise Karzinome des Respirations-, Reproduktions- und Gastrointestinaltraktes, sowie Tumore der Haut, der Brust und des vaskulären Systems, insbesondere aber das Mamma-Karzinom und das Cervix-Karzinom und seine Vorstufen, z.B. cervikale intraepitheliale Neoplasien (CIN I-III) sowie Karzinom in situ.

Der Ausdruck "automatisierbare Detektion" umfasst Verfahren, die die manuelle Arbeitskraft von menschlichem Personal ganz oder auch nur in Teilschritten ersetzt und insbesondere bei Schritten des Nachweisverfahrens oder bei der anschließenden Dokumentation oder Informationsverarbeitung Verwendung finden. Dies beinhaltet Schritte der Probenaufbereitung, Probenfärbung, Probenauslesung und Informationsverarbeitung. Die Detektion kann dabei sowohl über Absorptions-, Reflexions- oder Fluoreszenzmessungen erfolgen. Das Verfahren der Fluoreszenzmessung umfaßt sämtliche Methoden wie z.B. Fluoreszenzmikroskopie oder FACS (Durchflußzytometer)-Analyse.

Der Ausdruck "diagnostisches Expertensystem" umfasst eine Computersoftware, die die Bildinformationen in einen Diagnosevorschlag umwandelt. Dieses Expertensystem kann die gesamte vorhandene Information oder Teile davon aufgrund von in der Software vorhandenen Parametern oder von externen Informationen, auf die die Software zugreifen kann, zu einem Diagnosevorschlag konsolidieren. Sollten zur Absicherung des Diagnosevorschlags weitere Parameter erforderlich sein, kann die Software die Erhebung dieser Parameter vorschlagen oder durch Kopplung mit geeigneten Analysegeräten im Sinne eines Reflex-Algorithmus selbsttätig anfordern.

Der Ausdruck "Verstärkungssystem" umfasst biochemische Verfahren, die die Signalintensitäten erhöhen, so dass ein für den spezifischen Nachweis eines molekularen Markers günstigeres Signal/Rausch-Verhältnis erzeugt wird. Dies wird üblicherweise durch Einsatz von zusätzlichen Antikörpern und/oder enzymatischer Nachweisreaktionen erreicht.

Mit der vorliegenden Erfindung ist es möglich krankhaft veränderten Zellen, z.B. Karzinome und ihre Vorstufen spezifisch zu detektieren und die Herkunft von Metastasen zu bestimmen. Ein weiterer Gegenstand der Erfindung ist ein Kit zur Durchführung eines erfindungsgemäßen Verfahrens, das folgende Bestandteile enthält:
(a) Reagenzien zum Nachweis von mindestens zwei der molekularen Marker, nämlich markierte und/oder unmarkierte Antikörper gegen Her2/neu, p16, p53, Ki67, MN, mdm2, bcl2 und EGF-Rezeptor.
(b) Übliche Hilfsmittel, wie Puffer, Träger, Signalamplifikationssubstanzen, Färbereagenzien, etc.
(c) Automatisierte Verfahren der Probenaufbereitung, -färbung und Signaldetektion
(d) Protokolle und Reagentien zur Anfärbung von Zelllinien als Kontrollreaktion

Für die einzelnen Komponenten des Kits gelten die vorstehenden Ausführungen. Einzelne oder mehrere Komponenten des Kits können auch in veränderter Form Verwendung finden.

Mit der vorliegenden Erfindung ist es möglich, krankhaft veränderten Zellen, z.B. Karzinome und ihre Vorstufen manuell, aber auch in teil- oder vollautomatisierten Verfahren zu detektieren. Da die erfindungsgemäß erzielten Ergebnisse des gleichzeitigen Nachweises von mindestens zwei molekularen Markern nicht einer subjektiven Bewertung unterliegen, sondern vielmehr einer objektiven, automatisierten Detektion von krankhaften Veränderungen in biologischen Materialien förderlich sind, können die morphologischen Befunde durch objektive Parameter, auch in Form von "Reflex-Testing", ergänzt werden. Aufgrund der schnellen und automatisierten Handhabbarkeit der Verfahren sind sie für große, kosten- und personalsparende Screening-Verfahren geeignet. Darüber hinaus erlaubt die kombinatorische Färbung von mehr als zwei Markern die differentielle Diagnostik von krankhaften Veränderungen, sowie die Herkunftsbestimmung von Metastasen.

Somit stellt die vorliegende Erfindung einen wichtigen Beitrag zur modernen Diagnostik von Erkrankungen dar.

### Beispiele

Nachfolgend werden beispielhaft Protokolle zur Durchführung der beschriebenen Erfindung gegeben. In diesen Beispielen werden exakte Konditionen angegeben, die hier durch die Benutzung des Ventana Färbeautomaten NEXES vorgegeben sind. Das ändert nichts an der Tatsache, dass verschiedene Parameter, wie z.B. Inkubations- und Waschtemperatur, Inkubations- und Waschzeiten sowie die Konzentration von Antikörpern und anderen Reagenzien, variiert werden, d.h. an andere Geräte angepasst werden können. Ebenso können hier beschriebene Verstärkungssysteme weggelassen werden oder neu hinzugefügt werden, in Abhängigkeit von den jeweiligen Antikörpern bzw. DNA Sonden.

### Versuchsbeschreibung zum simultanen Nachweis von zwei molekularen Markern mit spezifischen Antikörpern auf Biopsien mit dem Immunfärbeautomaten Nexes der Firma Ventana

Zuerst werden von dem fixierten, in Paraffin eingebetteten Biopsiegeweben 4-5 µm dicke Paraffinschnitte (Mikrotom RM2155 von Leica) hergestellt und über Nacht bei 37°C getrocknet. Um ein Abschwimmen der Schnitte bei der späteren Färbung zu vermeiden, werden mit Silan beschichtete Objektträger benutzt. Zur Färbung werden die Paraffinschnitte 2 x 15 min. in Xylol entparaffiniert und anschließend durch eine absteigende Alkoholreihe (100 %, 90 %, 70 % je 2 x 5 min) geführt, um das Xylol wieder herauszuwaschen.

Durch die Fixierung mit Formalin entstehen Aldehydvernetzungen, die Antigene maskieren und somit für die Antikörper unzugänglich machen. Dadurch wird eine weitere Vorbehandlung notwendig. Die Demaskierung der Antigene erfolgt für 30 min in 10mM Citratpuffer, pH 6, bei 600 W in der Mikrowelle.

Nach der Mikrowellenvorbehandlung erfolgt die immunhistochemische Färbung im Nexes (Ventana Medical Systems) Immunfärbeautomaten. Alle Reagenzien werden bis auf die Primärantikörper und die Seren einzeln bzw. in Form von Kits über die Firma Ventana Medical Systems bezogen. Alle Inkubationen laufen bei 37°C ab. Der Färbeautomat wäscht automatisch nach jeder Inkubation der nachfolgend beschriebenen Reagenzien.

Zuerst erfolgt die Inaktivierung der endogenen Peroxidase mit dem im Kit beigefügten "Inhibitor" für 4 min. Anschließend werden mögliche unspezifische Bindungsstellen für 10 min mit 1,5 % Ziegenserum (Firma Vector) abgeblockt. Die Schnitte werden mit dem ersten monoklonalen Antikörper (her2/neu (Klon 3B5) von Oncogene Science/BAYER, 2,5 µg/ml in Ventana Antikörper-Verdünnungspuffer) für 30 min. inkubiert.

Um hinterher ein intensiveres Färbeergebnis zu bekommen, wird ein Verstärkungskit benutzt. Es besteht aus einem Kaninchen anti-Maus Immunglobulin, welches mit dem vorher gebundenen Primärantikörper reagiert und einem Maus anti-Kaninchen Immunglobulin, das wiederum an das vorherige Kaninchen anti-Maus Immunglobulin bindet. Die Inkubationszeit beträgt jeweils 8 min. Als nächstes wird ebenfalls für 8 min ein "MultiLink"-Antikörper eingesetzt, der sowohl gegen Maus, als auch gegen Kaninchen gerichtet und mit Biotin gekoppelt ist. Dieser bindet nun an alle drei vorher inkubierten Antikörper und vervielfacht die Biotinmenge an der Reaktionsstelle des Primärantikörpers. Als nächstes wird Streptavidin-HRP (Horse Radish Peroxidase) für 8 min zugegeben, welches an das Biotin bindet. Der Nachweis erfolgt durch Zugabe von DAB (Diaminobenzidin) und H₂O₂ als Substrate der Peroxidase, welches zu einem braunen Präzipitat umgesetzt wird.

Der Nachweis des zweiten Markers entspricht genau der oben beschriebenen Prozedur beginnend mit der Blockierung durch 1,5 % Ziegenserum und der Inkubation des zweiten spezifischen Primärantikörpers (p53, Klon DO1 von Oncogene Science/BAYER, 30 µg/ml in Ventana Antikörper-Verdünnungspuffer) für 30 min. Am Schluss wird anstelle des Streptavidin-HRP-Konjugats ein Streptavidin-ALP (Alkalische Phosphatase)-Konjugat verwendet. Nach dieser Inkubation wird die endogene alkalische Phosphatase mit Levamisole + MgCl₂ geblockt. Gleichzeitig wird auch das ALP Substrat (Fast Red und Naphtol) zugegeben. Anschließend erfolgt eine Kerngegenfärbung für 2 min mit Hematoxylin und danach werden die Schnitte 2 min in "Bluing Reagent" gefärbt. Zuletzt werden die Objektträger mit Moviol™ (Hoechst) eingedeckelt.

### Versuchsbeschreibung zur automatisierten Detektion abnormaler Zellen in einer Gewebeprobe, die durch simultanen Nachweis von zwei molekularen Markern angefärbt wurden

Biopsien, bei denen mindestens zwei molekulare Marker nach den oben beschriebenen Methoden mit Antikörpern nachgewiesen wurden, werden mittels eines Fluoreszenzmikroskops mit einem ansteuerbaren Kreuztisch für bis zu 8 Objektträger (Olympus AX70 mit Multicontrol-Box 2000-3 und analySIS Treibersoftware "Modul Stage^{m}"), einer modifizierten Version der analySIS 3.0 Software der Firma Soft Imaging Systems GmbH (SIS), die als SIS Paket "Grabbit Dual Pro" zusätzliche Module, insbesondere ein FFT- (="Fast Fourier Transformation"), ein MIA-(="Multiple Image Alignment") und ein C-Modul enthält, ausgewertet. Für die Bildaufnahmen wurden hochauflösende Schwarz-Weiß- (MV2 Slow Scan Kamera, 12 Bit von SIS) und Farbkameras (DXC-950P 3CCD Chip von Sony) verwendet. In Kombination sind diese Systeme für eine 16 Bit Grautonbildanalyse und zur Farbbildanalyse im RGB- und HSI-Farbraum bis 24 Bit Bildtiefe geeignet.

Die Position von insgesamt bis zu acht Objektträgern wird nach Kalibrierung des ansteuerbaren Kreuztisches (analySIS Modul Stage) durch Definition von acht aufeinanderfolgenden Tischwegen im Menü "Automation" (analySIS Modul Grains) relativ zum logischen Nullpunkt erfasst. Die Gesamtfläche der biologischen Proben wird flächendeckend in Einzelbereiche einer Größe von 158,7 um x 119,6 µm zerlegt, die dem Bildausschnitt eines Photos bei 40facher Vergrößerung mit der mikroskopischen Einheit entsprechen. Jede biologische Probe wird durch einen Tischweg analysiert. Jeder Tischweg wird meanderförmig horizontal als Serie von Tischwegpositionen abgefahren, so dass jeder Bereich der biologischen Probe der jeweiligen Objektträgerposition durch eine Tischwegposition "Stoß an Stoß" und nicht überlappend erfasst ist. Entsprechende Zeilenzahlen und Spaltenzahlen werden definiert. Die Addition der 8 Tischwege im Menü "Automation" ermöglicht die automatisierte Analyse aller auf dem Arbeitstisch positionierten Präparate.

Die Präparatanfärbungen jeder Tischwegposition werden im Durchlicht photographiert und die Bildinformationen im Menü "Oper" unter "Farbanpassung" auf "RGB angepasst". Dies ermöglicht die anschließende "Messung" im Menü "Automation". Für die Vermessung werden Schwellwerte für bis zu acht unterschiedliche Farbmischungen im Menü "Bilder" durch "Farbschwellwerte setzen" und Bestimmung von Pixelwerten mit "Pixelwerte/neu" definiert. Durch "Phasenanalyse" im Menü "Messen" werden die Einzelphotos der jeweiligen Tischwegpositionen an Hand der einmal definierten Farbschwellwerte vermessen und Bereiche bestimmter Farbmischungen als Fläche quantitativ in einer Excel-Datei wiedergegeben. Von besonderem Interesse sind beim gleichzeitigem Nachweis zweier Marker die Farben der Nachweisreaktionen, i.e. "Braun" (DAB) und "Rot" (FastRed) sowie deren Durchmischung ("Rotbraun") in einem Probenbereich. Somit erhält man für jeden einzelnen Tischweg (=Probe) eine Exceltabelle, wobei jede Spalte für die Flächen einer bestimmten Farbmischung steht. Die Zeilen der Exceltabelle entsprechen den Einzelphotos (=Tischwegpositionen) eines einzelnen Probenbereiches.

Mit einem Makro werden die Einzelwerte (=gemessene Fläche einer Farbmischung) einer Zeile (=Tischwegposition) jeweils mit einem definierten Schwellwert verglichen, der für die jeweilige Spalte bzw. Farbmischung vorgegeben wird. Das Makro verrechnet daraufhin die Einzelwerte, so dass die Probe als krank charakterisiert wird, wenn mehr als ein Einzelwert in einer Zeile über dem für die jeweilige Spalte definierten Schwellwert der Farbmischung liegt.

Die entsprechenden Bildpositionen können abgespeichert werden, so dass sie für eine spätere visuelle Begutachtung zur Verfügung stehen. Mit der Registrierkarte "Protokoll" im Menü "Automation" können Bilder und andere Daten für die jeweiligen Tischwegpositionen automatisch abgespeichert werden.

Diese Schritte lassen sich manuell ausführen, können aber auch automatisiert durchgeführt werden, indem Makros im Menü "Extras" unter "Makros aufzeichnen" erstellt und anschließend im Menü "Automation" abgerufen werden. Letztendlich erhält man für bestimmte Farbmischungen eine quantitative Aussage in Form einer Flächenangabe (= Fläche auf der biologischen Probe, die auf Grund der Anfärbung mit molekularen Markern genau definierte Mischfarben aufweist). Somit gelangt man über die quantitative Analyse der verrechneten Farbmischungen zu einer diagnostischen Aussage für die biologische Probe.

## Patentansprüche

1. Automatisierbares Verfahren zur Identifizierung von Krebszellen und deren Vorläufern, **dadurch gekennzeichnet, dass** mindestens zwei Marker in einer Zelle oder einer Gewebeprobe simultan detektiert werden und die Signalintensitäten kombiniert und verrechnet werden.

2. Verfahren nach Anspruch 1, **dadurch gekennzeichnet, dass** die automatische Informationsverarbeitung mit einem diagnostischen Expertensystem verbunden ist, das die Bildinformation zu einem Diagnosevorschlag konsolidiert.

3. Verfahren nach den Ansprüchen 1 bis 2, **dadurch gekennzeichnet, dass** die quantitative Erfassung der molekularen Marker durch Analyse von chromogenen Farbreaktionen oder Fluoreszenzsignalen in Teilbereichen der Gewebeprobe erfolgt, wobei Mischfarben bzw. die räumliche Nähe der Einzelfarben bei der Verwendung von mindestens zwei Markern zusätzliche Information gegenüber den Einzelfärbungen bietet.

4. Verfahren nach Anspruch 1 bis 4, **dadurch gekennzeichnet, dass** folgende Markerkombinationen detektiert werden:
Her2/neu und Ki67, Her2/neu und p53, her2/neu und bcl-2, her2/neu und MN,
her2/neu und mdm-2, her2/neu und EGF-Rezeptor,bcl-2 und Ki67, bcl-2 und
MN, bcl-2 und mdm-2, bcl-2 und EGF-Rezeptor, her2/neu und bcl-2,
P53 und bcl-2, p53 und MN, p53 und mdm-2, p53 und EGF-Rezeptor,
P16 und p53, p16 und MN, p16 und mdm-2, p16 und EGF-Rezeptor,
P16 und Ki67, P16 und her2/neu, P16 und bcl-2, MN und mdm-2, MN und
EGF-Rezeptor, mdm-2 und EGF-Rezeptor.

5. Verfahren nach Anspruch 1 bis 5, **dadurch gekennzeichnet, dass** Tumore der Brustdrüse, der Lunge, des Cervix, des Colon, der Haut und der Prostata detektiert werden.

6. Verfahren nach Anspruch 1 bis 5, **dadurch gekennzeichnet, dass** es sich um Reflex-Testing handelt.

7. Test Kit zur Durchführung des Verfahrens gemäß den Ansprüchen 1 bis6 enthaltend alle notwendigen Reagentien.
